Europäisches Patentamt

European Patent Office

Office européen des brevets

(11) Publication number: **0 430 414 A1**

# EUROPEAN PATENT APPLICATION

(21) Application number: 90311213.4

(22) Date of filing: 12.10.90

(51) Int. Cl.⁵: **A61K 31/485**, A61K 47/48, A61K 9/02

(30) Priority: 30.11.89 JP 309198/89

(43) Date of publication of application:
05.06.91 Bulletin 91/23

(84) Designated Contracting States:
AT BE CH DE DK ES FR GB GR IT LI LU NL SE
Bulletin 00/1

(71) Applicant: TORII & CO., LTD.
4-1, Nihonbashi-Honcho-3-chome
Chuo-ku Tokyo(JP)

(72) Inventor: Uekama,Kaneto
1716-80 Nagaminemachi,
Kumamoto-shi,(JP)
Inventor: Tanaka,Joji,
16-20-303,Nishikasai-3-chome,Edogawa-ku,
Tokyo,(JP)

(74) Representative: Silveston, Judith et al
ABEL & IMRAY Northumberland House
303-306 High Holborn
London, WC1V 7LH(GB)

(54) Morphine hydrochloride preparation for rectal administration.

(57) A hollow type suppository for rectal administration comprising a mixture of morphine hydrochloride with α-cyclodextrin, hydroxypropyl-α-cyclodextrin, dimethyl-β-cyclodextrin or hydroxypropyl-β-cyclodextrin.

FIG. I

MORPHINE CONCENTRATION IN PLASMA (ng/mL)

O : MORPHINE HYDROCHLORIDE : LACTOSE (1:100)

● : MORPHINE HYDROCHLORIDE : α-CYCLODEXTRIN (1:100)

TIME AFTER SUPPOSITORY ADMINISTRATION

EP 0 430 414 A1

# MORPHINE HYDROCHLORIDE PREPARATION FOR RECTAL ADMINISTRATION

The present invention relates to a novel preparation for rectal administration comprising a mixture of morphine hydrochloride with α-cyclodextrin, hydroxypropyl-α-cyclodextrin, dimethyl-β-cyclodextrin or hydroxypropyl-β-cyclodextrin.

Morphine hydrochloride, which is a representative of opium-group analgesics of strong action, is used for reliefing pains of end-stage cancer patients. The World Health Organization (WHO) has, in the WHO meeting on comprehensive therapy for cancer pains held in 1984, presented a pain treating method of WHO system and proposed, as the final means of reliefing the pain of cancerous pain patients in its three-step ladder of pain reliefing, the use of opium-group analgesics of strong action.

Morphine preparations available in Japan at present include (1) powders (original powder of morphine or 10-fold diluted powder), (2) tablets, (3) injections and (4) sustained release tablets. When peroral administration of morphine is difficult for such reasons as the site of cancer development, pathological conditions etc., morphine suppositories become necessary. Suppositories of morphine hydrochloride are not available on the market. In general, they are prepared at pharmacist's offices and administered, according to necessity.

The advantage of morphine suppository administration as compared with peroral administration lies in that the rectal administration is less susceptible to first pass effect; in other words, since the morphine absorbed does not pass through the liver because of vascularity, less proportion thereof is inactivated metabolically, so that the concentration of unchanged morphine distributed through the whole body is higher with statistical significance than that in peoral administration (Neil M: Ellison, Clinical Pharmacy, $\underline{3}$ - (6), 614, 1984).

Various attempts have been made to improve the release of morphine from the suppository thereof and thereby to improve the rise of its concentration in blood in rectal administration, in expectation of obtaining rapid efficacy. For example, mention may be made of morphine suppositories prepared by melt molding method from a mixture obtained by adding morphine hydrochloride to a glyceride of a saturated fatty acid (e.g., Witepsol® H15 and S55), cacao butter, and macrogol which are conventionally used suppository bases. However, no report can be found on the morphine concentration in blood in rectal administration of such suppositories.

Morphine suppositories of the prior art, including the above-mentioned morphine suppositories, are not satisfactory from the viewpoint of the release of morphine from the base and the storage stability of the preparation (cf., for example, Hiromi Torii et al., Kyushu-Yamaguchi Meeting of Japan Pharmaceutical Society, 1988). Further, since morphine hydrochloride is a narcotic, there is desired a suppository which can exhibit a sufficient analgesic effect by use of a small amount of morphine hydrochloride, in other words a suppository which exhibits enhanced bioavailability in rectal administration. However, no morphine suppository which is satisfactory in these points has been obtained yet.

The present inventors have made extensive study to solve the above problems and resultantly succeeded to create a hollow type suppository of morphine for rectal administration markedly improved in bioavailability and stability by mixing α-cyclodextrin, hydroxypropyl-α-cyclodextrin, dimethyl-β-cyclodextrin, or hydroxypropyl-β-cyclodextrin with morphine hydrochloride and filling the resulting mixture into a hollow type suppository. Thus, the present invention has been attained.

The hollow type suppository of the present invention can be prepared by known methods (cf., for example, Japanese Patent Application Kokai (Laid-open) No. 59-21,613, and Nobuaki Matsumoto et al., Shindan to Chiryo (Diagnosis and Therapy), 77 (4), 895-898, 1989) in the following manner.

Natural α-cyclodextrin obtainable by causing an enzyme to act on starch, or hydroxypropyl-α-cyclodextrin obtainable by chemical modification thereof, or dimethyl-β-cyclodextrin or hydroxypropyl-β-cyclodextrin, may be mixed with morphine hydrochloride in a proportion of 10-200 parts by weight, preferably 50-150 parts by weight, to 1 part by weight of morphine hydrochloride and the resulting mixture is filled into a hollow type suppository prepared with a conventional suppository base, whereby a hollow type suppository comprising morphine hydrochloride according to the present invention is obtained.

Alternatively, two or more cyclodextrins selected from α-cyclodextrin, hydroxypropyl-α-cyclodextrin, dimethyl-β-cyclodextrin and hydroxypropyl-β-cyclodextrin may be admixed with morphine hydrochloride and filled into a hollow type suppository for rectal administration according to the present invention.

In obtaining a hollow type suppository of the present invention, it is not necessary to form a complex of morphine hydrochloride with the cyclodextrin. It is only necessary to mix morphine hydrochloride with α-cyclodextrin, hydroxypropyl-α-cyclodextrin, dimethyl-β-cyclodextrin and/or hydroxypropyl-β-cyclodextrin and put the mixture in a hollow type suppository.

The hollow type suppository containing morphine hydrochloride for rectal administration according to the present invention shows promoted release, in particular promoted initial release, of morphine hydrochloride from the suppository, increased initial absorption velocity of morphine hydrochloride in the rectum and, further, enhanced bioavailability. Such an excellent effect can never be obtained with suppositories prepared by meltmolding morphine hydrochloride with a conventional suppository base or with hollow type suppositories prepared by filling morphine hydrochloride original powder alone; it is attributed to the improvement of absorption of morphine hydrochloride into the living body caused by the action on the mucous membrane structure or the surface active property of α-cyclodextrin, hydroxypropyl-α-cyclodextrin, dimethyl-β-cyclodextrin and hydroxypropyl-β-cyclodextrin. According to the study of the present inventors, such an effect was not obtained with other surface active agents than α-cyclodextrin, hydroxypropyl-α-cyclodextrin, dimethyl-β-cyclodextrin and hydroxypropyl-β-cyclodextrin.

The present invention provides α-cyclodextrin, hydroxypropyl-α-cyclodextrin, dimethyl-β-cyclodextrin or hydroxypropyl-β-cyclodextrin, or two or more thereof for admixture with morphine hydrochloride as the filling in a hollow type suppository.

The present invention also provides α-cyclodextrin, hydroxypropyl-α-cyclodextrin, dimethyl-β-cyclodextrin or hydroxypropyl-β-cyclodextrin, or two or more thereof in admixture with morphine hydrochloride as the filling in a hollow type suppository.

The present invention also provides the use of α-cyclodextrin, hydroxypropyl-α-cyclodextrin, dimethyl-β-cyclodextrin or hydroxypropyl-β-cyclodextrin, or two or more thereof, for the manufacture of a filled hollow type suppository containing morphine hydrochloride for the relief of pain.

The present invention also provides the use of α-cyclodextrin, hydroxypropyl-α-cyclodextrin, dimethyl-β-cyclodextrin or hydroxypropyl-β-cyclodextrin, or two or more thereof, in admixture with morphine hydrochloride in the manufacture of a filled hollow type suppository for the relief of pain.

The present invention also provides the use of α-cyclodextrin, hydroxypropyl-α-cyclodextrin, dimethyl-β-cyclodextrin or hydroxypropyl-β-cyclodextrin, or two or more thereof, to promote the release, in particular to promote the initial release, of morphine hydrochloride from a suppository, to increase the initial absorption velocity of morphine hydrochloride in the rectum and/or to enhance the bioavailability of morphine hydrochloride when administered rectally.

The present invention will be described further in detail below with reference to the Examples and Test Examples, which are not limiting.

Example 1

Preparation of hollow type suppository containing a morphine hydrochloride-α-cyclodextrin mixture

In an agate mortar were placed 100 mg of morphine hydrochloride and 10 g of α-cyclodextrin, then, a small amount of water was added thereto, and the resulting mixture was milled for about 1 hour. The milled product was dried under reduced pressure for 1 day, and the resulting solid mixture was seived through a No. 100 seive. As the control, a milled product was also prepared by using lactose in place of α-cyclodextrin. The hollow type suppository was prepared by using the Witepsol® H-15 base and with a 3 g suppository mold. The hollow part had an inside diameter of 5 mm and a space volume of about 0.5 cm$^3$. The intended suppository was prepared by filling in a hollow suppository 101 mg each (containing 1 mg of morphine hydrochloride) of the mixture of morphine hydrochloride with α-cyclodextrin or the mixture of morphine hydrochloride with lactose obtained above.

Example 2

Preparation of hollow type suppository containing a morphine hydrochloride-hydroxypropyl-α-cyclodextrin mixture

A hollow type suppository containing 1 mg of morphine hydrochloride was prepared in the same manner as in Example 1 except for using hydroxypropyl-α-cyclodextrin in place of α-cyclodextrin.

Example 3

Preparation of hollow type suppository containing a morphine hydrochloride-dimethyl-β-cyclodextrin mixture

A hollow type suppository containing 1 mg of morphine hydrochloride was prepared in the same manner as in Example 1 except for using dimethyl-β-cyclodextrin in place of α-cyclodextrin.

3

Example 4

Preparation of hollow type suppository containing a morphine hydrochloride-hydroxypropyl-$\beta$-cyclodextrin mixture

A hollow type suppository containing 1 mg of morphine hydrochloride was prepared in the same manner as in Example 1 except for using hydroxypropyl-$\beta$-cyclodextrin in place of $\alpha$-cyclodextrin.

Test Example 1

The present suppository and the control suppository prepared in Example 1 were respectively administered into the rectum of a native Japanese white rabbit weighing about 2 kg and the morphine concentration in the plasma of the rabbit was determined.

The rabbit was fasted for about 40 hours before administration, but given water ad libitum.

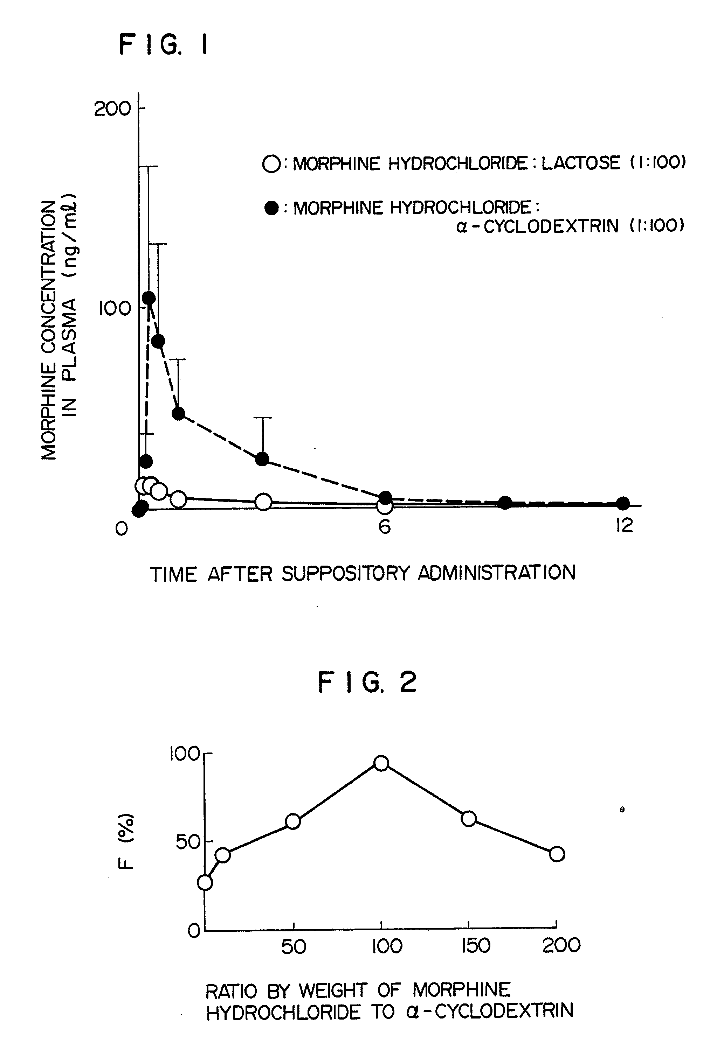

Each of the suppositories prepared in Example 1 was inserted into the rectum of the rabbit and the anus was pinched with a clip to prevent the leakage of the suppository ingredients. Blood samples were collected from the ear vein 0 minute, 5 minutes, 15 minutes, 30 minutes, 1 hour, 3 hours, 6 hours, 9 hours and 12 hours after the suppository administration, and the concentrations of morphine in the plasma were determined according to the method of Katagiri et al. (Journal of Pharmacy and Pharmacology, 40, 879-881 (1988)). The results are shown in Fig. 1. In the Figure, the solid line with circles represents the curve of the concentration in plasma after administration of the hollow type suppository of the control (containing 1 mg of morphine hydrochloride) filled with a mixture of morphine hydrochloride and lactose, while the dotted line with black circles represents that after administration of the present hollow type suppository filled with a mixture of morphine hydrochloride and $\alpha$-cyclodextrin (weight ratio = 1 : 100; containing 1 mg of morphine hydrochloride). As is apparent from the Figure, the present suppository shows good transfer of morphine into blood and exhibits a greatly increased morphine absorbability as compared with the control suppository, the maximum concentration in plasma (Cmax) being about 8 times and the AUC (area under the blood concentration-time curve) being about 5.8 times although the time required for reaching the Cmax (Tmax) being substantially the same.

Test Example 2

In the same manner as in Test Example 1, the hollow type suppositories prepared in Examples 2 and 3 (each containing 1 mg of morphine hydrochloride) were administered into the rectum of a rabbit, blood samples were collected 15 minutes, 30 minutes and 3 hours after the administration, and the morphine concentrations in blood were determined. The results are shown in Table 1. As compared with the control hollow type suppository (containing 1 mg of morphine hydrochloride) filled with a 1 : 100 mixture of morphine hydrochloride with lactose, the hollow type suppository filled with a 1 : 100 mixture of morphine hydrochloride with hydroxypropyl-$\alpha$-cyclodextrin (Example 2) or morphine hydrochloride with dimethyl-$\beta$-cyclodextrin (Example 3) gave a higher morphine concentration in blood and thus showed increased morphine absorption through the rectum when administered.

Table 1    Morphine concentration in rabbit plasma after suppository administration    (ng/ml)

|  | Time after rectal administration | | |
|---|---|---|---|
|  | 15 min. | 30 min. | 3 hrs. |
| Example 2 | 61 | 41 | 40 |
| Example 3 | 123 | 110 | 29 |
| Control a) | 14 | 8 | 3 |

Note: a)    Hollow type suppository filled with 1 : 100 mixture of morphine hydrochloride with lactose (containing 1 mg of morphine hydrochloride)

Test Example 3

In the same manner as in Example 1, hollow type suppositories were prepared which contained, relative to 1 mg of morphine hydrochloride, 0 mg (control suppository), 10 mg, 50 mg, 100 mg, 150 mg and 200 mg of $\alpha$-cyclodextrin. These suppositories were respectively administered into the rectum of a rabbit and the morphine concentrations in rabbit plasma were determined in the same manner as in Test Example 1. The results are shown in Fig. 2. As is apparent from Fig. 2, the suppositories according to the present invention show distinctly high bioavailability at the mixing ratio (weight ratio) of morphine hydrochloride to $\alpha$-cyclodextrin in the range of 1 : 10 to 1 : 200 as compared with the control suppository (containing no $\alpha$-cyclodextrin). In particular, the bioavailability in the range of said mixing ratio of 1 : 50 to 1 : 150 is more than two times that of the control.

BRIEF DESCRIPTION OF THE DRAWINGS

Fig. 1 is a graph showing the concentration in plasma exhibited when the present suppository (indicated by black circles) and the control suppository (indicated by circles) are administered to rabbits (n = 3).
Fig. 2 is a graph showing the effect of the mixing ratio of morphine hydrochloride to $\alpha$-cyclodextrin on the bioavailability of morphine. The F(%) indicated as the ordinate of the graph represents the bioavailability of the present suppository, the AUC (area under the blood concentration-time curve) of the morphine concentration in plasma exhibited when 1 mg of morphine hydrochloride is intravenously administered to a rabbit being taken as 100.

**Claims**

1. A hollow type suppository for rectal administation containing a mixture of morphine hydrochloride with one or more cyclodextrins selected from $\alpha$-cyclodextrin, hydroxypropyl-$\alpha$-cyclodextrin, dimethyl-$\beta$-cyclodextrin and hydroxypropyl-$\beta$-cyclodextrin.

2. A suppository according to claim 1 wherein the weight ratio of morphine hydrochloride to the cyclodextrin(s) is within the range of from 1 : 10 to 1 : 200.

3. A suppository according to claim 2 wherein the weight ratio of morphine hydrochloride to the cyclodextrin(s) is within the range of from 1 : 50 to 1 : 150.

4. α-Cyclodextrin, hydroxypropyl-α-cyclo-dextrin, dimethyl-β-cyclodextrin or hydroxypropyl-β-cyclodextrin or two or more thereof, for admixture with morphine hydrochloride as the filling for the manufacture of a filled hollow type suppository for the relief of pain.

5. α-Cyclodextrin, hydroxypropyl-α-cyclo-dextrin, dimethyl-β-cyclodextrin or hydroxypropyl-β-cyclodextrin or a mixture of two or more thereof in admixture with morphine hydrochloride as the filling for the manufacture of a filled hollow type suppository for the relief of pain.

6. Use of α-cyclodextrin, hydroxypropyl-α-cyclodextrin, dimethyl-β-cyclodextrin or hydroxypropyl-β-cyclodextrin or two or more thereof for the manufacture of a filled hollow type suppository containing morphine hydrochloride for the relief of pain.

7. Use of α-cyclodextrin, hydroxypropyl-α-cyclodextrin, dimethyl-β-cyclodextrin or hydroxypropyl-β-cyclodextrin or two or more thereof in admixture with morphine hydrochloride for the manufacture of a filled hollow type suppository for the relief of pain.

8. Use of α-cyclodextrin, hydroxypropyl-α-cyclodextrin, dimethyl-β-cyclodextrin or hydroxypropyl-β-cyclodextrin or two or more thereof to promote the release of morphine hydrochloride from a supppository, to increase the intial absorption velocity of morphine hydrochloride form the rectum and/or to enhance the bioavailability of morphine hydrochloride when administered rectally.

CLAIMS for the contracting states ES, GR:

1. A process for the production of a filled hollow type suppository for rectal administation comprising mixing morphine hydrochloride with one or more cyclodextrins selected from α-cyclodextrin, hydroxypropyl-α-cyclodextrin, dimethyl-β-cyclodextrin and hydroxypropyl-β-cyclodextrin and filling the resulting mixture into a hollow type suppository.

2. A process according to claim 1 wherein the weight ratio of morphine hydrochloride to the cyclodextrin-(s) is within the range of from 1 : 10 to 1 : 200.

3. A process according to claim 2 wherein the weight ratio of morphine hydrochloride to the cyclodextrin-(s) is within the range of from 1 : 50 to 1 : 150.

4. Use of α-cyclodextrin, hydroxypropyl-α-cyclodextrin, dimethyl-β-cyclodextrin or hydroxypropyl-β-cyclodextrin or two or more thereof for the manufacture of a filled hollow type suppository containing morphine hydrochloride for the relief of pain. 5. Use of α-cyclodextrin, hydroxypropyl-α-cyclodextrin, dimethyl-β-cyclodextrin or hydroxypropyl-β-cyclodextrin or two or more thereof in admixture with morphine hydrochloride for the manufacture of a filled hollow type suppository for the relief of pain.

7. Use of α-cyclodextrin, hydroxypropyl-α-cyclodextrin, dimethyl-β-cyclodextrin or hydroxypropyl-β-cyclodextrin or a mixture of two or more thereof to promote the release of morphine hydrochloride from a supppository, to increase the intial absorption velocity of morphine hydrochloride form the rectum and/or to enhance the bioavailability of morphine hydrochloride when administered rectally.

## F I G. I

MORPHINE CONCENTRATION IN PLASMA (ng/mℓ)

○ : MORPHINE HYDROCHLORIDE : LACTOSE (1:100)

● : MORPHINE HYDROCHLORIDE : α-CYCLODEXTRIN (1:100)

TIME AFTER SUPPOSITORY ADMINISTRATION

## F I G. 2

F (%)

RATIO BY WEIGHT OF MORPHINE HYDROCHLORIDE TO α-CYCLODEXTRIN

7

European
Patent Office

**EUROPEAN SEARCH
REPORT**

Application Number

**EP 90 31 1213**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | J. SZEJTLI: "Cyclodextrin technology", 1988, pages 54-57,186-189,283-285, Kluwer, Dordrecht, NL<br>* Pages 55-56,187-188,283-285 * | 8 | A 61 K<br>31/485<br>A 61 K 47/48<br>A 61 K 9/02 |
| Y | IDEM | 1-7 | |
| Y | FILE SERVER STN, FILE CHEMICAL ABSTRACTS, vol. 105, no. 12, abstract no. 102482n, Columbus, Ohio, US; Y. WATANABE et al.: "Pharmaceutical evaluation of hollow type suppositories. IV. Improvement of bioavailability of propranolol in rabbits after rectal administration",<br>& J. PHARMACOBIO-DYN., 9(6), 526-31<br>* Whole abstract * | 1-7 | |
| D,Y | CLINICAL PHARMACY, vol. 3, November-December 1984, pages 614-617, Bethesda, MD, US; N.M. ELLISON et al.: "Plasma concentrations following single doses of morphine sulfate in oral solution and rectal suppository"<br>* Page 616, paragraph 1: "Discussion" * | 1-8 | |
| D,Y | EP-A-0 094 157 (TAKEDA CHEMICAL INDUSTRIES LTD)<br>* Page 16, line 13 - page 19, line 5; examples 20-28 * | 1-8 | **TECHNICAL FIELDS SEARCHED (Int. Cl.5)**<br><br>A 61 K |
| A | FILE SERVER STN, FILE MEDLINE, accession no. 90106730, Derwent Publications Ltd, London, GB; Y. MAT-SUMOTO et al.: "Enhancing effect of glyceryl-1-monooctanoate on the rectal absorption of gentamicin from hollow-type suppositories in rabbits",<br>& CHEM. PHARM. BULL. (TOKYO), (1989 SEP) 37 (9) 2477-80<br>* Whole abstract * | | |

−/−

The present search report has been drawn up for all claims

| Place of search | Date of completion of search | Examiner |
|---|---|---|
| The Hague | 14 February 91 | SITCH W.D.C. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

& : member of the same patent family, corresponding
document

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | FILE SERVER STN, FILE BIOSIS, accession no. 90:356525 & BIOLOGICAL ABSTRACTS, abstract no. 90:53104, Derwent Publications Ltd, London, GB; Y. MATSUMOTO et al.: "Therapeutic management of terminal cancer pain by morphine rectal delivery", & YAKUZAIGAKU 50 (1). 1990. 53-57 * Whole abstract * | | |
| P,A | DATABASE WPI(L), accession no. 90-080964 [11], Derwent Publications Ltd, London, GB; & JP-A-2 036 123 (DAITO KOEKI K.K.) * Whole abstract * | | |

TECHNICAL FIELDS SEARCHED (Int. Cl.5)

The present search report has been drawn up for all claims

| Place of search | Date of completion of search | Examiner |
|---|---|---|
| The Hague | 14 February 91 | SITCH W.D.C. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document